# EUROPÄISCHE PATENTSCHRIFT

(11) **EP 1 295 124 B1**
(45) Veröffentlichungstag und Bekanntmachung des Hinweises auf die Patenterteilung: **18.02.2004**
(21) Anmeldenummer: 01962796.7
(22) Anmeldetag: 29.06.2001
(51) Int. Cl.: G01N 33/543, G01N 33/53, G01N 33/542, G01N 33/58

(54) **KOMPETITIVES ASSAY-VERFAHREN**
COMPETITIVE ASSAY METHOD
PROCEDE D'ESSAI COMPETITIF

(30) Priorität: 29.06.2000 DE 10030798
(43) Veröffentlichungstag der Anmeldung: 26.03.2003
(73) Patentinhaber: Evotec Technologies GmbH, 40225 Düsseldorf (DE)
(72) Erfinder: MEYER-ALMES, Franz-Josef, 58636 Iserlohn (DE)
(74) Vertreter: Meyers, Hans-Wilhelm, Dr.Dipl.-Chem.
(86) Internationale Anmeldenummer: PCT/EP2001/007424
(87) Internationale Veröffentlichungsnummer: WO 2002/001226

(56) Entgegenhaltungen:
- EP-A- 0 105 714
- EP-A- 0 411 945
- EP-A- 0 726 464
- WO-A-93/14404
- WO-A-95/15498
- GB-A- 2 084 317

## Beschreibung

Die Erfindung betrifft ein Verfahren zum Nachweis der Bindung und zur Bestimmung der Bindungseigenschaften von Molekülen, insbesondere von Liganden an korrespondierende Rezeptormoleküle in einem homogenen und kompetitiven Untersuchungsverfahren sowie ein Testverfahren zur Ermittlung des Einflusses von synthetischen oder biologischen Substanzen auf die Bindungseigenschaften von Molekülen.

Rezeptor-Bindungs-Untersuchungsverfahren, wie beispielsweise immunologische Nachweisverfahren, haben in der in vitro Diagnostik und in der präklinischen Forschung in Testsystemen zur Auffindung von Leitstrukturen für die Wirkstoffentwicklung eine hohe Bedeutung erlangt. Ursache ist, dass sie hochspezifisch und äußerst empfindlich sind und zudem im Zytoplasma gelöste oder auf Zellmembranen befindliche Rezeptoren bedeutende Mediatoren von intrazellulären oder extrazellulären biologischen Einflüssen sind. Als Beispiele für die Vielzahl solcher Rezeptoren sind Rezeptoren für Neurotransmitter wie z.B. Acetylcholin, Endorphine und Enkephaline; Rezeptoren für Wachstumsfaktoren wie z.B. Epidermis-Wachstumsfaktor (EGF), Platelet-derived Growth Faktor (PDGF) und Nervenwachstumsfaktor (NGF); und Rezeptoren für Steroidhormone, wie z.B. Östrogene, Progesteron, Glucocorticoide, zu nennen. Die Bindung der Liganden an ihren jeweiligen Rezeptor kann z.B. zur Auslösung einer Kaskade enzymatischer Reaktionen, wie z.B. der Adenylat-Cyclase-Kaskade, der Phosphoinosititkaskade oder einer Beeinflussung der Tyrosin-Kinase-Aktivität ihrer Rezeptoren, führen und so verschiedene Prozesse in der Zelle beeinflussen. Insbesondere Steroidhormon-Rezeptorkomplexe sind auch in der Lage in die Kerne von Zellen einzudringen und dort die Transkription verschiedener Gene direkt zu beeinflussen. Eine Störung beispielsweise dieser biologischen Regelsysteme ist oft die Ursache für Krankheiten, wie z.B. Diabetes, Krebs oder neurologische Erkrankungen.

Es ist daher wünschenswert, Verfahren zur spezifischen und schnellen Diagnose zu haben. Zudem ist es notwendig durch Testverfahren Substanzen zur Behandlung solcher Krankheiten zu finden. Es sind z.B. Substanzen geeignet, die direkt in die Signaltransduktionsprozesse in einer Zelle oder zwischen Zellen eingreifen, wobei unter Signaltransduktionsprozessen allgemein die Weitergabe eines Signals verstanden wird. Sei es beispielsweise durch die Auslösung einer enzymatischen Reaktion, einer Kaskade enzymatischer Reaktionen, durch Änderung der Ionenstärke oder Ionenzusammensetzung einer Zelle oder durch andere Prozesse. Diese Substanzen können beispielsweise durch Bindung an den Rezeptor, das von diesem ausgehende Signal verstärken (sog. Agonisten) und/oder durch Bindung an den Rezeptor die Signaltransduktion blockieren (sog. Antagonisten).

Diese Nachweisverfahren beruhen auf der Wechselwirkung zwischen den Rezeptoren und ihren jeweiligen natürlichen Bindungspartnem (Liganden). In Testsystemen wird der Einfluss von synthetischen oder biologischen Substanzen auf die Bindungseigenschaften der Uganden zu diesen Rezeptoren untersucht. Es sind hierzu bereits mehrere verschiedene Gruppen von Bestimmungsmethoden bekannt.

WO-A-9 515 498 bezieht sich auf Immunoassaymethoden zur Bestimmung des Anteils eines Analyten in einer Probe, durch direkte Kompetition eines Hapten-Analyt-Konjugates mit dem Analyten um die Bindung an einen anti-Analyt Antikörper. Hierbei ist das Hapten-Analyt-Konjugat an einen anti-hapten Antikörper gebunden.

Bei einigen Methoden wird die direkte Bindung zwischen markiertem Ligand und Rezeptor und/oder Ligand und markiertem Rezeptor bestimmt. Andere Verfahren beruhen auf der Kompetition von bindungsaktiven Substanzen mit dem markierten Liganden um deren Bindung an den Rezeptor.

Beispielsweise wird in immunologischen Sandwich-Assays der Analyt von zwei verschiedenen Antikörpern gebunden. Einer der beiden Antikörper trägt eine Markierung, wodurch seine Konzentration bestimmt werden kann. Im Falle kleiner Analyte finden in der Regel kompetitive Assay-Verfahren Anwendung. Dabei konkurrieren der Analyt und ein synthetisches Derivat des Analyten um die Bindungsstellen des Antikörpers. Markiert wird entweder der Antikörper oder das Analytderivat.

Die Markierung kann dabei aus einem radioaktiven Isotop, einem nichtradioaktiven Isotop, einem Chromophor, einem Fluorophor, Edelmetallkolloiden (wie z.B. Gold, Silber), mit Biopolymeren, mit synthetischen Polymeren, Biotin, Peptiden mit Knäuel-Struktur (Coil-Coil-Interaction), repetitiven Histidinresten (zur Bindung an Ni-NTA), Enzymen, einem spontan oder induzierbar chemoluminiszierenden oder phosphoreszierenden Molekül, einem Molekül, dessen Elektrolumineszenz induziert werden kann, oder Seltenerdechelaten bestehen.

Oftmals sind die Untersuchungssysteme heterogen, d.h. ein Bindungspartner muss vor der Messung aus der Reaktionslösung abgetrennt werden. Ein Beispiel für diese Systeme sind heterogene Rezeptor-Radioligand-Assays. Zur Bestimmung der Bindung von Substanzen an Rezeptoren, wird hier die an den Rezeptor gebundene Radioaktivität (Rezeptor-Ligand-Komplex) gemessen und ins Verhältnis zur Gesamtaktivität gesetzt. Dazu ist es notwendig, vor der Messung der Aktivität des Rezeptor-Ligand-Komplexes diesen von freiem Liganden zu trennen. Dies kann beispielsweise physikalisch durch Gelfiltration, Polyethylenglykol-Fällung, Adsorption an Polyethylenimin-getränkte Filter, Adsorption an DEAE-Filter, Adsorption an Aktivkohle, Zentrifugation, Dialyse oder Gelelektrophorese erfolgen (Receptor-Effector Coupling-A Practical Approach, Edited by E.C. Hulme, The Practical Approach Series, Series Editors: D. Rickwood and B.D. Hames, IRL Press, Oxford University Press, Oxford 1990, M. Salomonsson et al. J. Steroid Biochm. Molec. Biol. 50, 313-18 (1996), P.V.M. Shekhar et al. J. of the National Cancer Institute 89, 1774-1782 (1997)). Als Voraussetzung für seine Anwendbarkeit muss, aufgrund des notwendigen Abtrennungsschrittes, die Dissoziationsgeschwindigkeit der Ligand-Rezeptor-Komplexe vernachlässigbar gegenüber dem Zeitbedarf für die Trennung sein. Andernfalls würde nur noch ein Bruchteil des Komplexes bestimmt werden können und damit die Affinität unterbewertet.

Bei homogenen Untersuchungsverfahren entfällt der Abtrennungsschritt, wie das folgende Beispiel eines homogenen radioaktiven Assays zeigt. Beim sogenannten "Szintillation Proximity Assay" (SPA) (Nichols JS et al. Anal. Biochem, 1998, 257, 112-9; Park YW; Anal. Biochem., 1999, 269, 94-104; Horton JK and Baxendale PM, Methods Mol Biol, 1995, 41, 91-105) werden die zu untersuchenden Rezeptoren an szintillationsfähige Polymerkügelchen gebunden. Die zu untersuchenden Substanzen werden mit Tritium markiert. Ein meßbares Szintillationssignal wird nur dann erzeugt, wenn eine markierte Substanz an einen Rezeptor bindet.

Allerdings gilt für alle radiometrischen Testmethoden, dass der Einsatz von radioaktiven Isotopen ein hohes Gefährdungspotential aufweist und die Entsorgungskosten sehr hoch sind, daher werden z.B. Fluoreszenzfarbstoffe an Stelle von radioaktiven Isotopen eingesetzt. Wobei sich vergleichbare Sensitivitäten bei einer Verkürzung der Messzeiten erreichen lassen.

Beispiele für verschiedene Fluoreszenzmethoden sind im folgenden ausgeführt:
- Fluoreszenzenergietransfer: Dieses Verfahren basiert auf dem Fluoreszenzenergietransfer zwischen einem Donor- und einem Akzeptorfluoreszenzfarbstoffmalekül. Für die sogenannte "Time-resolved fluorescent assay technology" (HTRF) wird als Donor ein Seltenerdekryptat-Komplex und als Akzeptor z.B. Allophycocyanin eingesetzt. Dazu müssen beide Bindungspartner mit entweder einem Donor- oder Akzeptormolekül markiert werden (US Patent 5,527,684; US Patent 5,512,493; Mathis G. Clin. Chem. 39:9 (1993); Kolb A., Burke J., Mathis G., Editor J. Devlin, 345-360, Marcel Dekker, New York (1997); Mathis G., Clin. Chem. 41:9 (1995)).
- Fluoreszenzpolarisation: Als weiteres Assayprinzip hat sich sowohl im Bereich der medizinischen Diagnostik als auch im Bereich der pharmazeutischen Wirkstoffsuche die Fluoreszenzpolarisation etabliert (Onnerfjord P; et al. J. Immunol Methods, 1998 Apr, 213:1, 31-9; OConnell MT;et al. Ther Drug Monit, 1995 Oct, 17:5, 549-55, Saunders NJ; et al. J Antimicrob Chemother, 1995 Aug, 36:2, 411-5). Die Methode erlaubt die Unterscheidung zwischen kleinen fluoreszenten Liganden und deren Komplexen mit Rezeptoren allerdings nur, falls die Rezeptormoleküle eine deutlich größere Masse als die Liganden aufweisen, wobei diese zur Erreichung guter Messergebnisse eine wesentlich geringere Masse als 20 kDa haben sollten. Fluoreszenzkorrelationsspektroskopie: Ein weiteres Verfahren ist die Fluoreszenzkorrelationsspektroskopie, die bereits in den Bereichen medizinische Diagnostik und Wirkstoffsuche eingesetzt wird (Koltermann A; et al. Proc Natl Acad Sci U S A, 1998 Feb, 95:4, 1421-6; Sterrer S; et al. J Recept Signal Transduct Res, 1997 Jan, 17:1-3, 511-20; Auer M et al., Drug Discovery Today, 1998, 3: 457-465). Diese Methode basiert ebenfalls auf der Unterscheidung von Molekülen hinsichtlich ihrer Molekularmassendifferenzen. Es lassen sich Bindungsgrade von kleinen Molekülen an großen Komplexen bzw. Rezeptoren bestimmen, ohne das bei Fluoreszenzintensitätsmessungen häufig auftretende Problem des abnehmenden Signal/Rausch-Verhältnisses bei kleiner werdendem Assayvolumen. Zudem ist kein sogenannter "Propellereffekt" zu beobachten, der eine auftretende Fluoreszenzpolarisation depolarisieren kann.
- Fluorescence Intensity Distribution Analysis (FIDA): Diese Methode erlaubt es Moleküle hinsichtlich Ihrer spezifischen Heiligkeiten zu unterscheiden. Sie ist zudem molekulargewichtsunabhängig und erlaubt extrem kurze Messzeiten von wenigen Sekunden (PCT/EP97/05619; Kask et al. 1999 Procl. Natl. Sci. USA. 96,13756).

Als weitere Verfahren sollen noch die Elektrochemoluminiszenz sowie die sogenannte Fluorometric microvolume assay technoloy (FMAT) erwähnt werden.
- Elektrochemilumineszenz: In US Patents 5945344, 5858676, 5804400, 5798083, 5705402, 5714089 wird eine Methode für die Quantifizierung von Bindungsreaktionen u.a. auch Rezeptor-Ligand-Interaktionen beschrieben, die auf der Elektrochemoluminiszenz von Rutheniumchelatkomplexen an der Oberfläche von Elektroden beruht. Allerdings findet hier die signalgenerierende Reaktion an einer Elektrodenoberfläche statt, so dass Oberflächenartefakte auftreten können.
- FMAT: Die Fiuorometric Microvolume Assay Technology (FMAT)-Methode verwendet ganze Zellen oder Mikropartikel, auf deren Oberfläche sich die Rezeptoren befinden. Mit einem fokussierten Laserstrahl wird eine Fläche mit immobilisierten Zellen/Mikropartikeln abgetastet und so die Bindung von fluoreszenten Liganden an die Rezeptoren gemessen. (Swartzman et al., Anal. Biochem. 1999, 271, 143-151)

Eine Voraussetzung der Anwendbarkeit aller dieser bisher bekannten Untersuchungsmethoden ist allerdings, dass sowohl der unmarkierte native Ligand als auch sein markiertes Konjugat dieselben oder nahezu identische Bindungseigenschaften aufweisen. Da Ligand und Rezeptor aufgrund des Schlüssel-Schloßprinzips sehr spezifisch miteinander wechselwirken, werden oftmals, insbesondere wenn sterisch anspruchsvolle Markierungen verwendet werden, nur noch der unmarkierte Ligand spezifisch gebunden, die markierten Derivate hingegen zeigen veränderte Bindungseigenschaften, wobei oftmals gar keine Bindung zum korrespondierenden Rezeptor stattfindet.

Insbesondere bei der Durchführung kompetitiver Assays bereitet die Wahl des markierten Konjugates bzw. Ligandderivates mit geeigneter Affinität zum Rezeptormolekül häufig Probleme. Diese ergeben sich daraus, dass die Affinität des Ligandderivates zum Rezeptormolekül in einem bestimmten Verhältnis zur Affinität des Liganden zum Rezeptormolekül stehen muss. Ist beispielsweise die Affinität des Ligandderivaten zum Rezeptormolekül zu hoch, so wird das Reaktionsgleichgewicht sehr in Richtung des Rezeptormolekül-Ligand-Derivats verschoben und so das Verfahren ungenau, bzw. wenig aussagekräftig. Vorinkubation des Liganden mit dem Rezeptormolekül oder eine chemische Modifizierung des Derivates können die Aussagekraft des Verfahrens verbessern, sind aber oft mit Schwierigkeiten verbunden und insbesondere die chemische Modifizierung oft nicht durchführbar. Vergleichbares gilt, wenn die Affinität des Ligandderivaten zum Rezeptormolekül zu niedrig ist.

Ein Beispiel für einen Ligand Rezeptor-Komplex, der aufgrund dieses Problems den bekannten Assayverfahren nicht zugänglich ist, ist die Bindung von Estrogen an den humanen Estrogen-Rezeptor alpha (ERα). Es sind bis jetzt nur wenige fluoreszente Estrogenderivate bekannt, die überhaupt eine Bindung an den ERα zeigen. Allerdings sind diese Derivate für den Einsatz in einem Assay-Verfahren nicht geeignet, da diese Derivate entweder keine spezifische Bindung an den Rezeptor ausbilden und somit die Affinität des Estrogenderivates an den Rezeptor nur sehr gering ist oder ihre Synthese ist sehr aufwendig und daher sehr zeit- und insbesondere kostenintensiv (Nehci I., et al. J. of Histochemistry and Cytochemistry, 1980, 28:1081-88, Joyce BG, et al. 1982, 18:1147-55, French AN, et al. Steroids, 58:157-168; Bindal RD et al. J. steroid. Biochem., 1987, 28:361-370; Carlson KE, et al. 1989, 32:345-355). Vergleichbares gilt für die Bindung von weiteren Hormonen, wie beispielweise Testosteron, Progesteron oder Androgen oder auch für die Bindung von Peptid-Liganden, wie beispielsweise Somatostatin an ihre jeweiligen Rezeptoren.

Die Aufgabe der vorliegenden Erfindung bestand nun darin, einen Rezeptor-Bindungsassay zu entwickeln, der die vorstehenden Nachteile überwindet. Der insbesondere die Bestimmung von Bindungsparametem für die Bindung von Rezeptoren an Liganden ermöglicht, von denen keine geeigneten markierten Konjugate bekannt sind, die vom korrespondierenden Rezeptor spezifisch und hochaffin, d.h; mit Bindungsparametern vergleichbar zum unmarkierten Uganden, gebunden werden.

Die Aufgabe wird erfindungsgemäß mit Hilfe eines kompetitiven Assay-Verfahrens mit folgenden Schritten gelöst:
- Herstellung einer Lösung mit mindestens zwei miteinander in Wechselwirkung tretenden Molekülverbänden (**A, B**), die jeweils aus mindestens 2 unterscheidbaren Molekülen (A, A'; B, B') bestehen, wobei die Bindung mindestens zweier Moleküle der Lösung die Änderung mindestens einer zu detektierenden Eigenschaft bewirkt,
- Bestimmung der mindestens einen zu detektierenden Eigenschaft.

Ferner umfasst die Erfindung ein Testverfahren zur Ermittlung des Einflusses von synthetischen oder biologischen Substanzen auf die Bindungseigenschaften von Molekülen in Molekülverbänden, mit folgenden Schritten:
- Herstellung einer Lösung mit mindestens zwei miteinander in Wechselwirkung tretenden Molekülverbänden (**A, B**), die jeweils aus mindestens 2 unterscheidbaren Molekülen (A, A'; B, B') bestehen, wobei die Bindung mindestens zweier Moleküle der Lösung die Änderung mindestens einer zu detektierenden Eigenschaft bewirkt,
- Zugabe eines Analyten
- Bestimmung der mindestens einen zu detektierenden Eigenschaft.

Molekülverbände im Sinne der vorliegenden Erfindung sind chemische Bindungsgleichgewichtssysteme. Wobei die Moleküle eines Molekülverbandes mindestens einen Ligand und/oder ein Ligandderivat und mindestens einen korrespondierenden Rezeptor umfassen.

Der Begriff "Ligand" im Sinne dieser Erfindung bezeichnet jedes Molekül, das durch mindestens ein Molekül gebunden werden kann. Erfindungsgemäß kann es sich hierbei um DNA-Moleküle und/oder RNA-Moleküle und/oder kleine organische Moleküle, Kohlenhydrate, Medikamente, insbesondere Hormone, . beispielsweise Steroidhormone oder Peptidhormone, Neurotransmitter, Steroide und/oder Makromoleküle, insbesondere verbrückte Käfigstrukturen, handeln. Es können aber auch Peptide, Proteine, Antigene, virale Antigene und/oder Wachstumsfaktoren sein.

Der Begriff "Rezeptormolekül" oder "Rezeptor" bezeichnet jedes Molekül, das eine spezifische Bindungsstelle für mindestens ein Molekül aufweist. Insbesondere handelt es sich hierbei um Peptide und/oder Proteine insbesondere Antikörper, Rezeptoren, virale Rezeptoren, G-Protein gekoppelte Rezeptoren. Es können erfindungsgemäß auch DNA-Moleküle und/oder RNA-Moleküle und/oder kleine organische Moleküle, insbesondere Steroide, Kohlenhydrate, Medikamente und/oder Makromoleküle, insbesondere verbrückte Käfigstrukturen, sein.

Erfindungsgemäß ist die Einteilung der Moleküle in Ligand und Rezeptor willkürlich und dient lediglich zur besseren Veranschaulichung des erfindungsgemäßen Verfahrens. Beispielsweise kann die Unterscheidung der Moleküle über ihre Größe erfolgen. Daher kann ein Molekül, das in einem Bindungsgleichgewichtssystem als Ligand bezeichnet wird, in einem weiteren Bindungsgleichgewichtssystem als Rezeptor bezeichnet werden.

Der Begriff "Ligandderivat" oder "Konjugat" bezeichnet eine Substanz, die durch chemische Modifizierung des Liganden, insbesondere durch Versehen des Liganden mit einer zu detektierenden Eigenschaft, erhalten wird.

Der Begriff "Analyt" bezeichnet eine synthetische oder biologische Substanz oder eine Kombination davon, deren Bindungseigenschaften an einen Rezeptor der Lösung untersucht werden sollen. Beispielsweise kann es sich dabei um Peptide, Proteine, DNA-Moleküle, RNA-Moleküle, kleine organische Moleküle, insbesondere Steroide, Kohlenhydrate, Medikamente und/oder Makromoleküle, insbesondere verbrückte Käfigstrukturen, handeln.

Es ist erfindungsgemäß wünschenswert, dass mindestens ein Molekülverband der Lösung keine zu detektierende Eigenschaft aufweist somit mindestens einen Rezeptor (B) und einen Liganden (B') aufweist und mindestens ein weiterer Molekülverband der Lösung eine zu detektierende Eigenschaft aufweist somit mindestens aus einem Rezeptor (A) und einem Ugandderivat (A'), das eine zu detektierende Eigenschaft aufweist, besteht, wobei sich bevorzugt die Rezeptoren der Molekülverbände untereinander unterscheiden. Erfindungsgemäß bedeutet der Begriff "keine zu detektierende Eigenschaft", dass der Molekülverband **B** überhaupt keine detektierbare Eigenschaften aufweist, sondern keine der im tatsächlich durchgeführten Assayverfahren gemessenen detektierbaren Eigenschaften besitzt. Damit interferiert dann der Molekütverband **B** nicht mit dem von Molekülverband **A** gelieferten Messsignale.

Erfindungsgemäß umfasst die mindestens eine zu detektierende Eigenschaft die Markierung mit mindestens einem radioaktiven Isotop, einem nichtradioaktiven Isotop, einem Chromophor, einem Fluorophor, einem Edelmetallkolloid, einem Biopolymer, einem synthetischen Polymer, Biotin, einem Peptid, einer Kette von Histidinresten, einem Enzym, einem chemilumineszenten Molekül, einem phosphoreszierenden Molekül, einem Molekül, das zur Elektrochemilumineszenz angeregt werden kann, einem Seltenerdechelat oder einer Kombination davon.

Zur Bestimmung der mindestens einen detektierbaren Eigenschaft eignen sich beispielsweise radiometrische Methoden oder fluorimetrische Methoden. Besonders bevorzugt sind erfindungsgemäß fluorimetrische Methoden, wie konfokale Fluoreszenzspektroskopie, Fluoreszenz-Korrelationsspektroskopie und/oder Fluorescence Intensity Distribution Analysis (FIDA). Wobei als Parameter beispielsweise Translationsdiffusionsgeschwindigkeit, Rotationsgeschwindigkeit, Lebensdauer angeregter Zustände, Polarisation von Strahlung, Energietransfer, Quantenausbeute, Partikelzahl, Konzentration und/oder Intensitätsdifferenz ermittelt werden können.

Weiterhin ist es bevorzugt, dass mindestens ein Molekül (B oder B'), insbesondere ein Rezeptor, mindestens eines Molekülverbandes, der keine zu detektierende Eigenschaft aufweist, nur an mindestens ein Molekül (B oder B'), insbesondere Liganden, seines keine zu detektierende Eigenschaft aufweisenden Molekülverbandes bindet.

Es ist ebenfalls vorteilhaft, dass mindestens ein Molekül (A'), insbesondere ein Ligandderivat, mindestens eines Molekülverbands, eine zu detektierende Eigenschaft aufweist und nur an mindestens ein Molekül (A), z.B. einen Rezeptor, seines eine zu detektierende Eigenschaft aufweisenden Molekülverbands bindet.

Wobei in einer weiteren bevorzugten Ausführungsform das mindestens eine Molekül (A), insbesondere ein Rezeptor, mindestens eines Molekülverbandes sowohl an mindestens ein, eine zu detektierende Eigenschaft aufweisendes Molekül (A'), z.B. ein Ligandderivat, seines Molekülverbandes als auch an mindestens ein Molekül (B oder B'), insbesondere einen Liganden, mindestens eines weiteren Molekülverbandes, der keine zu detektierende Eigenschaft aufweist, bindet.

Die efindungsgemäßen Verfahren werden bevorzugt unter Verwendung von genau zwei miteinander in Wechselwirkung tretenden Molekülverbänden durchgeführt.

Im Falle von zwei in Wechselwirkung tretenden Molekülverbänden besteht somit ein Molekülverband, der keine zu detektierende Eigenschaften aufweist, aus mindestens einem Liganden (B') und mindestens einem ersten Rezeptor (B), der nur den mindestens einen Liganden (B') binden kann und der andere Molekülverband aus mindestens einem Ligandderivat (A') und mindestens einem weiteren Rezeptor (A), der sowohl den Liganden (B') als auch das Ugandderivat (A'), das eine zu detektierende Eigenschaft aufweist, bindet. Wobei sich bevorzugt die mindestens zwei Rezeptoren (A, B) der jeweiligen Molekülverbände unterscheiden.

Bei der Durchführung des erfindungsgemäßen Verfahrens ist es wünschenswert, dass die Konzentration des mindestens einen Moleküls (A) eines Molekülverbandes, das sowohl das mindestens eine Molekül (A') seines Molekülverbandes, das eine zu detektierende Eigenschaft aufweist als auch mindestens ein Molekül weiterer Molekülverbände ohne diese Eigenschaften (B') bindet und die Konzentrationen dieser Moleküle (A', B') mit oder ohne zu detektierender Eigenschaften so aufeinander abgestimmt sind, dass diese mindestens zwei Moleküle (A', B') miteinander um die Bindung an das mindestens eine Molekül (A) eines Molekülverbandes konkurrieren.

Die Abstimmung der Konzentration von A' und B' kann beispielsweise über die Berücksichtigung der Bindungskonstanten der Moleküle A' bzw. B' zu A oder B erfolgen.

Darüber hinaus ist es vorteilhaft, die Konzentration des mindestens einen Moleküls (B) eines Molekülverbandes, das nur das mindestens eine Molekül (B'), das keine zu detektierenden Eigenschaften besitzt bindet, so zu bemessen, dass die Kompetitionswirkung des mindestens einen Moleküls (B'), das keine zu detektierende Eigenschaften besitzt, gegenüber mindestens einem Molekül (A '), das eine zu detektierende Eigenschaft aufweist, um die Bindung an das mindestens eine Molekül (A) eines Molekülverbandes ganz oder teilweise aufgehoben wird.

Wobei es bevorzugt ist, dass die Bindung des mindestens einen Moleküls (A'), das eine zu detektierende Eigenschaft aufweist, an das mindestens eine Molekül (A), durch eine Änderung dieser Eigenschaft angezeigt wird.

Zur Bestimmung der Bindungsparameter von Molekülen in Molekülverbänden kann es erfindungsgemäß daher wünschenswert sein, dass die Konzentrationen des mindestens einen Rezeptors (A) eines Molekülverbandes, der sowohl Ligandderivate (A'), die eine zu detektierende Eigenschaft aufweisen, seines Molekülverbandes als auch mindestens einen Liganden (B') mindestens eines weiteren Molekülverbandes, der keine zu detektierenden Eigenschaften besitzt, bindet und die Konzentrationen dieser Liganden und Ligandderivate (A', B') so aufeinander abgestimmt sind, dass diese mindestens zwei Moleküle (A', B') miteinander um die Bindung an den mindestens einen Rezeptor (A) eines Molekülverbandes konkurrieren. Wobei es erfindungsgemäß bevorzugt ist, dass die Bindung des mindestens einen Rezeptors (A) an das mindestens eine Ligandderivat (A'), das eine zu detektierende Eigenschaft aufweist, durch eine Änderung dieser Eigenschaft angezeigt wird. In Gegenwart mindestens eines Rezeptors (B), der nur mindestens einen Liganden (B') bindet, der keine zu detektierenden Eigenschaften aufweist, wird die Kompetition des mindestens einen Liganden (B') und des mindestens einen Ligandderivats (A') um die Bindung zum mindestens einen Rezeptor (A) ganz oder teilweise aufgehoben, was zu einer Änderung der Anzahl der Ligandderivat (A')-Rezeptor (A)-Komplexe führt und in einer Änderung der zu detektierenden Eigenschaft angezeigt wird.

Zur Bestimmung des Einflusses von synthetischen oder biologischen Substanzen auf die Bindungseigenschaften von Molekülen in Molekülverbänden ist es erfindungsgemäß wünschenswert, dass die Konzentrationen des mindestens einen Rezeptors (A) eines Molekülverbandes, der sowohl mindestens ein Ligandderivat (A'), das eine zu detektierende Eigenschaft aufweiset, seines Molekülverbandes als auch mindestens einen Liganden (B'), der keine zu detektierenden Eigenschaften aufweist, mindestens eines weiteren Molekülverbandes bindet und die Konzentrationen dieser Liganden und Ligandderivate (A', B') so aufeinander abgestimmt sind, dass diese mindestens zwei Moleküle (A', B') miteinander um die Bindung an den mindestens einen Rezeptor (A) eines Molekülverbandes konkurrieren. Darüber hinaus ist erfindungsgemäß die Konzentration des mindestens einen Rezeptors (B) eines Molekülverbandes, der nur den Liganden (B') bindet, so zu bemessen, dass die Kompetitionswirkung des mindestens einen Liganden (B'), der keine zu detektierende Eigenschaften besitzt gegenüber dem mindestens einen Ligandderivat (A'), das eine zu detektierende Eigenschaft aufweist, um die Bindung an den mindestens einen Rezeptor (A) eines Molekülverbandes, ganz oder teilweise aufgehoben wird. Die Gegenwart eines Analyten, der an den mindestens einen Rezeptor (B) eines Molekülverbandes bindet, kann eine Freisetzung des Liganden (B') bewirken, welcher dann mit dem Ligandderivat (A') um die Bindung an den Rezeptor (A) konkurriert, was durch eine Änderung der zu detektierenden Eigenschaften angezeigt wird.

Erfindungsgemäß ist es daher wünschenswert, dass der Analyt an das mindestens eine Molekül (B) eines Molekülverbandes bindet, der nur Moleküle bindet, die keine zu detektierende Eigenschaft besitzen.

Darüber hinaus kann es erfindungsgemäß vorteilhaft sein, dass mindestens ein Molekül eines Molekülverbandes an eine Oberfläche gebunden ist, insbesondere an Oberflächen von Trägerpartikeln, Mikro- bzw. Nanotiterplatten, Polymerpartikeln, fluoreszenzmarkierte Polymerpartikeln. Wobei in einer bevorzugten Ausführungsform die Trägerpartikel aus Metalloxiden, Silikat, Metallcolloid, Silberhalogenid, Arsensulfid, Nickelsulfid, Cobaldsulfid, Kohlenstoffcolloid und/oder organischem Polymer insbesondere verzweigtem Polymer wie z.B. Dendrimere bestehen.

In einer weiteren Ausführungsform des erfindungsgemäßen Verfahrens kann es bevorzugt sein, die Bindung des Analyten durch Zugabe von weiteren mit einer zu detektierenden Eigenschaft versehenden Molekülen nachzuweisen, insbesondere kann dies in Form eines ELISA-Verfahrens oder Immunoblottings durchgeführt werden.

Insgesamt eignet sich das erfindungsgemäße Verfahren besonders zur Bestimmung der Bindungseigenschaften von Molekülen, die durch das Einbringen einer zu detektierenden Eigenschaft ihre Bindungseigenschaften ändern oder sogar ihre Bindungsfähigkeit ganz verlieren und darüber hinaus zur Identifizierung von Analyten insbesondere von Antikörpern, Nukleinsäuren, Proteinen, Peptiden, Hormonen.

Ein Beispiel für diese Ausführungsform des erfindungsgemäßen Verfahrens soll im folgenden anhand des oben beschriebenen Beispiels der Bindung von Estradiol an den humanen Estradiol-Rezeptor alpha (ERa) näher erläutert werden.

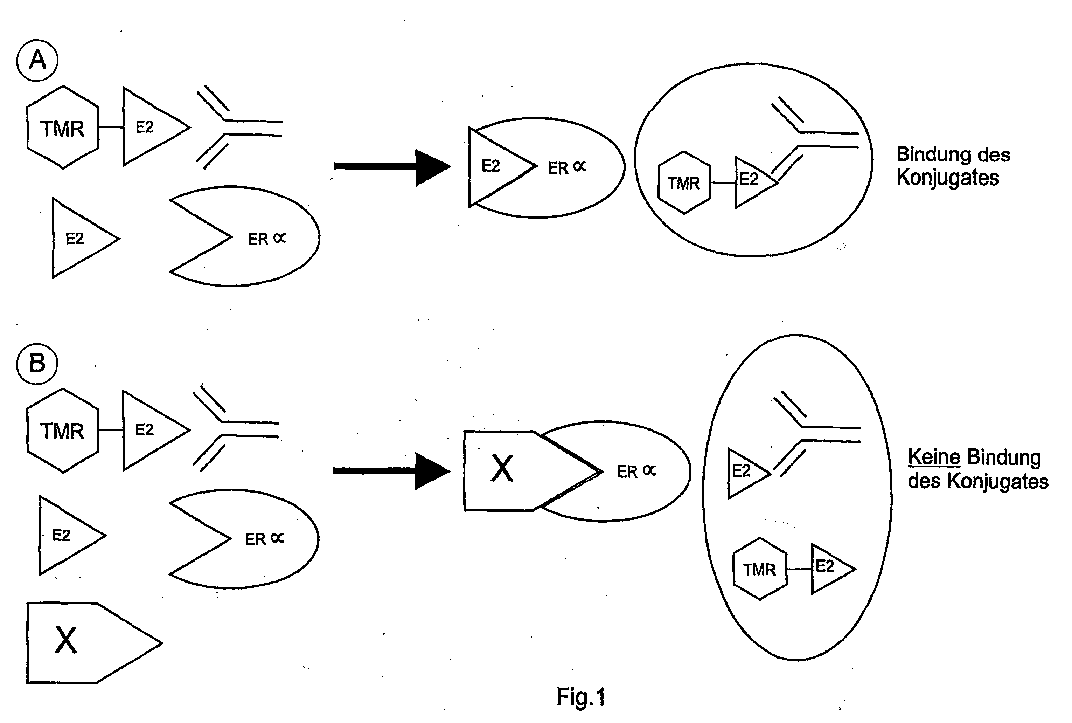

Ein Schema dieser besonderen Ausführungsform des erfindungsgemäßen Assayverfahrens ist in Abbildung 1 dargestellt.

Zunächst wird eine Lösung aus A = anti-Estradiol-Antikörper, A'= TAMRA-Estradiol (TMR-E2), B = Rezeptor ERα und B' = Estradiol (E2) hergestellt. Die Konzentrationen von Estradiol (E2), TAMRA-Estradiol (TMR-E2), welches nicht an den Rezeptor ERα bindet, ERα und anti-Estradiol-Antikörper sind so zu bemessen, dass das Estradiol vollständig an den ERα bindet und nur TMR-E2 an den anti-Estradiol-Antikörper, der sowohl TMR-E2 als auch E2 bindet, bindet kann (Abbildung 1A).

Enthält die Lösung oder wird zu der Lösung nun Substanz X (Analyt) gegeben, deren Bindungseigenschaften an den Rezeptor ERα untersucht werden sollen, so hat diese Substanz im Falle einer Nichtbindung an den Rezeptor keinen Einfluss auf das Bindungsgleichgewichtssystem. Bindet diese Substanz aber, wie in Abbildung 1B dargestellt, an den Estradiol-Rezeptor ERα, so wird durch diese Bindung E2 freigesetzt. Dieses E2 konkurriert dann mit TMR-E2 um die Bindung an den Antikörper. Dies bewirkt letztendlich eine Freisetzung des TMR-E2-Konjugats, die durch eine Änderung der TMR-Fluoreszenz bestimmt werden kann.

Die Bestimmung der TMR-Fluoreszenz erfolgt besonders vorteilhaft durch fluorimetrische Methoden, wie konfokaler Fluoreszenzspektroskopie, Fluoreszenz-Korrelationsspektroskopie und dem FIDA-Verfahren. Wobei sich Translationsdiffusionsgeschwindigkeit, Rotationsgeschwindigkeit, Lebensdauern angeregter Zustände, Polarisation von Strahlung, Energietransfer, Quantenausbeute, Partikelzahl oder Konzentration und/oder Intensitätsdifferenz ermitteln lassen.

### Beispiel 1

### Estradiol-Assay

### Verwendete Materialien:

### 17-β-Estradiol (Sigma), 4-Hydroxytamoxifen (Sigma), Estrogenrezeptor-alpha, human recombinant (Panvera), anti-Estradiol-Antikörper (Biotrend), Streptavidin-funktionalisierte Nanopartikel (Miltenyl), TMR-Estradiol

### Herstellung von TAMRA-Estradiol:

5 ml einer Lösung von 55 mg 6-Ketoestradiol-(O-carboxymethyl)-oxim und 18 mg N-Hydroxy-succinimid (Merck) wurden in 5 ml Methanol gelöst und mit 24 µl N,N'-diisopropylcarbodiimid (Fluka) versetzt. Die Lösung wurde für 2 h bei 25°C gerührt. Anschließend wurde über einen Zeitraum von 30 min eine Lösung von 115 mg 1,8-Diamino-3,6-dioxaoctan (Merck) in Methanol tropfenweise hinzugefügt. Nach der Zugabe von 2,4 µl N,N'-diisopropylcarbodiimid wurde die Lösung erneut für 16 h bei 25°C gerührt. Das Lösungsmittel wurde dann im Vakuum entfernt und durch Flash -Chromatographie auf Silica-Gel 60 (Merck) ein Amino-funktionales Estradiol-Derivat gewonnen. Zur Herstellung von TMR-Estradiol wurden 1 mg dieses Derivats in 50 µl Dimethylformamid (DMF, Fluka) gelöst und anschließend eine Lösung aus 1.1 mg 5-Carboxytetramethylrhodamin-Succinimidyl-Ester in 200 µl DMF zugegeben. Die Lösung wurde für 16 h gerührt und anschließend das Lösungsmittel im Vakuum entfernt. TMR-Estradiol wurde dann durch RP-HPLC aufgereinigt und spektroskopisch charakterisiert (MALDI ToF: berechnete Masse: 902, gefundene Masse: 903 [M+H]+, UV/Vis: λₘₐₓ= 558 nm und Fluoreszenzspektroskopie: λ_{exc}= 543 nm, λₑₘ= 588 nm)

### Assaypuffer:

PBS-Puffer (8 g NaCl (Merck), 0.2 g KCl (Merck), 1.44 g Na₂HPO₄ (Merck), 0.24 g KH₂PO₄ (Merck) pro Liter H₂O; pH 7.4) versetzt mit 1.5 mM EDTA, 3 mM MgCl₂, 0.05% Pluronic F-127 (Sigma), 2 mM DTT, pH 7.5

### Durchführung:

25 nM 17-β-Estradiol in Assaypuffer und jeweils unterschiedliche Konzentrationen von 4-Hydroxytamoxifen (0,11 nM; 0,22nM; 0,44 nM; 0,88nM; 1,75nM; 3,51nM; 7,02nM; 9,38nM; 14,04nM; 18,76nM; 18,8nM; 28,14nM; 37,6nM; 56,28nM; 112,56nM; 225,12nM; 450nM; 1000nM) wurden gemischt und zusammen mit 40 nM Estrogenrezeptor alpha (PANVERA) (in Assaypuffer) für 10 min bei 25°C inkubiert. Anschließend wurde ein Gemisch aus 5 nM TAMRA-Estradiol and 2 nM biotinyliertem anti-Estradiol-Antikörper (jeweils in Assaypuffer) hinzugegeben und für weitere 10 Minuten bei 25°C inkubiert. Nach Zugabe von 50 pM Streptavidin-funktionalisierter Nanopartikel und erneuten 5 min Inkubationszeit bei 25°C erfolgte die Bestimmung der Bindung von TAMRA-Estradiol an den Antikörper durch das nachfolgend beschriebene FIDA-Experiment bei 543 nm.

### FIDA-Messungen

Hierzu wurde eine konfokales Mikroskop "ConfoCor" (Carl Zeiss, Jena-EVOTEC BioSystems AG, Hamburg) mit Hardware zur Aufnahme der Anzahl der von der zu untersuchenden Lösung pro Zeiteinheit emittierten Photonen ausgerüstet.

Pro Messung wurden jeweils 15 µl der zu untersuchenden Lösung auf die Glasoberfläche einer Nunc-Kammer pipettiert und der Focus 150 µm oberhalb der Oberfläche eingestellt. Der Focus wurde durch eine Spiegelanordnung mit einer Frequenz von 25 Hz und einer Amplitude von 100 µm abgelenkt. Während der Messung (Dauer ca. 2-5 s) wurde der Probentisch um 500 µm verschoben, so dass insgesamt eine Fläche von 500 x 100 µm vermessen werden konnte.

Die Fluoreszenz des in den zu untersuchenden Lösungen enthaltenden Fluoreszenzfarbstoffes (Tetramethylrhodamin, TAMRA) wurde bei λ = 543 nm mit Hilfe eines 1.5 mW Helium-Neon Lasers angeregt. Die Laserintensität wurde dabei durch Filter so eingestellt, dass der Anteil an Triplett-Fluoreszenz einer 1 nM TMR-Lösung kleiner als 15% war. Unter diesen Bedingungen hatte TMR eine molekulare Helligkeit von 30 bis 40 kHz. Streulicht wurde durch einen 590 DF40 Filter im Detektionsstrahlengang abgetrennt. Zur Photonenaufzeichnung diente eine Avalanche-Photodiode. Es wurden jeweils die Photonen pro Zeitintervall aufgezeichnet.

Durch Anpassen der erstellten Histogramme (Photonen/Zeit) mittels bestimmter Modelle (Kask et al. 1999, Proc. Natl. Sci. USA. 96, 13756) wurden Werte für die molekulare Helligkeit und die Konzentration des freien und des gebundenen TAMRA-Estradiols in der Lösung erhalten. Zur graphischen Darstellung der Parameter wurde das Programm Origin 5.0 (Microcal) verwendet. Für jede Lösung wurden 10 Messungen zu jeweils 2 Sekunden durchgeführt um Mittelwerte und Standardabweichungen zu bestimmen.

### Ergebnis:

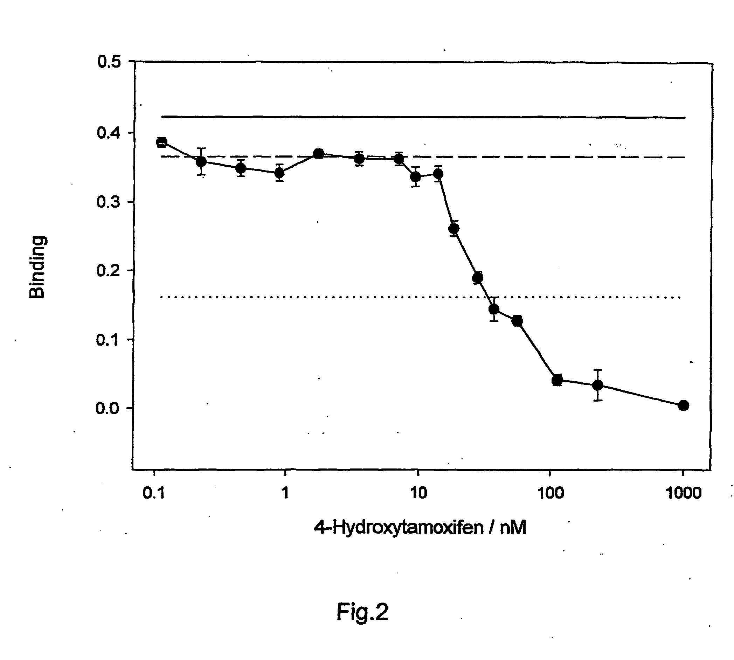

Mit zunehmender Konzentration an 4-Hydroxytamoxifen wurde immer mehr Estradiol freigesetzt, welches dann mit TAMRA-Estradiol um die Bindung an den Antikörper konkurrierte. Infolge der zunehmenden Kompetition nahm das Signal des an den Antikörper gebundenen TAMRA-Estradiols ab (Abbildung 2).

## Patentansprüche

1. Kompetitives Assayverfahren zur Bestimmung der Bindung von Molekülen, mit folgenden Verfahrensschritten:
a) Herstellen einer Lösung mit mindestens zwei miteinander in Wechselwirkung tretenden Molekülverbänden (**A, B**), die jeweils aus mindestens 2 unterscheidbaren Molekülen (A, A'; B, B') bestehen,
wobei der Molekülverband **B** nur aus Motekülen (B, B') besteht, die keine zu detektierende Eigenschaft aufweisen,
und dass mindestens ein Molekül (B oder B') des mindestens einen Molekülverbands **B**, nur an mindestens ein Molekül (B oder B') dieses keine zu detektierende Eigenschaft aufweisenden Molekülverbands **B** bindet,
wobei die Bindung mindestens zweier Moleküle der Lösung die Änderung mindestens einer zu detektierenden Eigenschaft bewirkt,
b) Bestimmung der mindestens einen zu detektierenden Eigenschaft, wobei die Molekülverbände chemische Bindungsgleichgewichtssysteme sind, und die Moleküle eines Molekülverbands mindestens einen Ligand und/oder ein Ligandderivat und mindestens einen korrespondierenden Rezeptor umfassen.

2. Verfahren gemäß Anspruch 1, **dadurch gekennzeichnet, dass** zur Ermittlung des Einflusses von synthetischen oder biologischen Substanzen auf die Bindungseigenschaften von Molekülen in Molekülverbänden zu der Lösung ein Analyt zugegeben wird.

3. Verfahren gemäß Anspruch 1 und/oder 2, **dadurch gekennzeichnet, dass** mindestens ein Molekül (A) des mindestens eines Molekülverbandes A sowohl an mindestens ein eine zu detektierende Eigenschaft.aufweisendes Molekül (A') seines Molekülverbandes **A** als auch an mindestens ein Molekül (B', B) mindestens des mindestens weiteren Molekülverbandes **B**, der keine zu detektierende Eigenschaft aufweist, bindet.

4. Verfahren gemäß mindestens einem der Ansprüche 1 bis 3, **dadurch gekennzeichnet, dass** mindestens ein Molekül (A') des mindestens eines Molekülverbands **A** eine zu detektierende Eigenschaft aufweist und nur an mindestens ein Molekül (A) dieses eine zu detektierende Eigenschaft aufweisenden Molekülverbands **A** bindet.

5. Verfahren gemäß mindestens einem der Ansprüche 1 bis 4, **dadurch gekennzeichnet, dass** die Konzentrationen des mindestens einen Moleküls (A) des Molekülverbandes **A**, das sowohl das mindestens eine Molekül (A') seines Molekülverbandes **A**, das eine zu detektierende Eigenschaft aufweist als auch mindestens ein Molekül weiterer Molekülverbände **B** ohne diese Eigenschaften (B') bindet und die Konzentrationen dieser Moleküle (A', B') mit oder ohne zu detektierender Eigenschaften so aufeinander abgestimmt sind, dass diese mindestens zwei Moleküle (A', B') miteinander um die Bindung an das mindestens eine Molekül (A) des Molekülverbandes **B** konkurrieren.

6. Verfahren gemäß mindestens einem der Ansprüche 1 bis 5, **dadurch gekennzeichnet, dass** die Konzentration des mindestens einen Moleküls (B) eines Molekülverbandes **B**, das nur das mindestens eine Molekül (B'), das keine zu detektierende Eigenschaften besitzt bindet, so bemessen ist, dass die Kompetitionswirkung des mindestens einen Moleküls (B'), das keine zu detektierende Eigenschaften besitzt, gegenüber mindestens einem Molekül (A'), das eine zu detektierende Eigenschaft aufweist, um die Bindung an das mindestens eine Molekül (A) des Molekülverbandes A ganz oder teilweise aufgehoben wird.

7. Verfahren gemäß mindestens einem der Ansprüche 1 bis 6, **dadurch gekennzeichnet, dass** die Bindung des mindestens einen Moleküls (A'), das eine zu detektierende Eigenschaft aufweist, an das mindestens eine Molekül (A), durch eine Änderung dieser Eigenschaft angezeigt wird.

8. Verfahren gemäß mindestens einem der Ansprüche 1 bis 7, **dadurch gekennzeichnet, dass** der Analyt an das mindestens eine Molekül (B) eines Molekülverbandes B bindet, der nur Moleküle bindet, die keine zu detektierende Eigenschaft besitzen.

9. Verfahren gemäß mindestens einem der Ansprüche 1 bis 8, **dadurch gekennzeichnet, dass** die mindestens eine detektierbare Eigenschaft durch die Markierung mindestens eines Moleküls mit einem radioaktiven Isotop, einem nichtradioaktiven Isotop, einem Chromophor, einem Fluorophor, einem Edelmetallkolloid, einem Biopolymer, einem synthetischen Polymer, Biotin, einem Peptid, einer Kette von Histidinresten, einem Enzym, einem chemilumineszenten Molekül, einem phosphoreszierenden Molekül, einem Molekül, das zur Elektrolumineszenz angeregt werden kann, einem Seltenerdechelat oder einer Kombination davon erzeugt wird.

10. Verfahren nach mindestens einem der Ansprüche 1 bis 9, **dadurch gekennzeichnet, dass** die Bestimmung der mindestens einer detektierbaren Eigenschaft mittels radiometrischer Methoden und/oder fluorimetrischer Methoden erfolgt.

11. Verfahren nach mindestens einem der Ansprüche 1 bis 10, **dadurch gekennzeichnet, dass** aus den detektierbaren Eigenschaften Parameter ermittelt werden.

12. Verfahren nach mindestens einem der Ansprüche 1 bis 11, **dadurch gekennzeichnet, dass** die Moleküle Peptide, Proteine, G-Protein gekoppelte Rezeptoren, DNA-Moleküle, RNA-Moleküle, Makromoleküle sind.

13. Verfahren gemäß mindestens einem der Ansprüche 1 bis 12, **dadurch gekennzeichnet, dass** mindestens ein Molekül eines Molekülverbandes an eine Oberfläche gebunden ist.

14. Verfahren gemäß Anspruch 13, wobei die Trägerpartikel aus Metalloxiden, Silikat, Metallcolloid, Silberhalogenid, Arsensulfid, Nickelsulfid, Cobaldsutfid, Kohlenstoffcolloid und/oder organischem Polymer bestehen.

15. Verfahren gemäß mindestens einem der Ansprüche 1 bis 14, **dadurch gekennzeichnet, dass** die Bindung des Analyten durch Zugabe von weiteren mit einer zu detektierenden Eigenschaft versehenden Molekülen nachgewiesen werden kann.

16. Verwendung des Verfahrens gemäß mindestens einem der Ansprüche 1 bis 15 zur Bestimmung der Bindungseigenschaften von Molekülen, die durch das Einbringen einer zu detektierenden Eigenschaft ihre Bindungseigenschaften ändern.

17. Verwendung des Verfahrens gemäß mindestens einem der Ansprüche 1 bis 16 zur Identifizierung von Analyten.

## Claims

1. A competitive assay method for determining the binding of molecules, comprising the following process steps:
a) preparing a solution with at least two mutually interacting molecular aggregates (**A, B**) each of which consists of at least 2 distinguishable molecules (A, A'; B, B'), wherein molecular aggregate **B** exclusively consists of molecules (B, B') which do not have a detectable property, wherein at least one molecule (B or B') of said at least one molecular aggregate **B** exclusively binds to at least one molecule (B or B') of said molecular aggregate **B** having no detectable property, wherein the binding of at least two molecules of the solution causes the change of at least one detectable property;
b) determining said at least one detectable property;
wherein said molecular aggregates are chemical binding equilibrium systems and the molecules of one molecular aggregate comprise at least one ligand and/or a ligand derivative and at least one corresponding receptor.

2. The method according to claim 1, **characterized in that** an analyte is added to the solution for establishing the influence of synthetic or biological substances on the binding properties of molecules in molecular aggregates.

3. The method according to claim 1 and/or 2, **characterized in that** at least one molecule (A) of said at least one molecular aggregate **A** binds to both at least one molecule (A') of its molecular aggregate **A** having a detectable property and at least one molecule (B', B) of at least said at least one further molecular aggregate **B** having no detectable property.

4. The method according to at least one of claims 1 to 3, **characterized in that** at least one molecule (A') of said at least one molecular aggregate **A** has a detectable property and exclusively binds to at least one molecule (A) of said molecular aggregate **A** having a detectable property.

5. The method according to at least one of claims 1 to 4, **characterized in that** the concentrations of said at least one molecule (A) of molecular aggregate **A** which binds to both said at least one molecule (A') of its molecular aggregate **A** having a detectable property and at least one molecule of further molecular aggregates **B** having no such property (B') and the concentrations of these molecules (A', B') having or not having properties to be detected are matched to one another so that said at least two molecules (A', B') compete for the binding to said at least one molecule (A) of the molecular aggregate **B**.

6. The method according to at least one of claims 1 to 5, **characterized in that** the concentration of said at least one molecule (B) of a molecular aggregate **B** which exclusively binds said at least one molecule (B') having no detectable property is such that the competitive action of said at least one molecule (B') having no detectable property towards at least one molecule (A') having a detectable property for the binding to said at least one molecule (A) of molecular aggregate **A** is completely or partially neutralized.

7. The method according to at least one of claims 1 to 6, **characterized in that** the binding of said at least one molecule (A') having a detectable property to said at least one molecule (A) is indicated by a change of this property.

8. The method according to at least one of claims 1 to 7, **characterized in that** the analyte binds to said at least one molecule (B) of a molecular aggregate **B** which exclusively binds molecules having no detectable property.

9. The method according to at least one of claims 1 to 8, **characterized in that** said at least one detectable property is produced by labeling at least one molecule with a radioactive isotope, a non-radioactive isotope, a chromophore, a fluorophore, a noble metal colloid, a biopolymer, a synthetic polymer, biotin, a peptide, a chain of histidine residues, an enzyme, a chemiluminescent molecule, a phosphorescent molecule, a molecule which can be excited to electroluminescence, a rare-earth metal chelate, or a combination thereof.

10. The method according to at least one of claims 1 to 9, **characterized in that** the determination of said at least one detectable property is effected by radiometric methods and/or fluorimetric methods.

11. The method according to at least one of claims 1 to 10, **characterized in that** parameters are established from said detectable properties.

12. The method according to at least one of claims 1 to 11, **characterized in that** said molecules are peptides, proteins, receptors coupled to G protein, DNA molecules, RNA molecules, macromolecules.

13. The method according to at least one of claims 1 to 12, **characterized in that** at least one molecule of a molecular aggregate is bound to a surface.

14. The method according to claim 13, wherein the support particles consist of metal oxides, silicate, metal colloid, silver halide, arsenium sulfide, nickel sulfide, cobalt sulfide, carbon colloid and/or organic polymer.

15. The method according to at least one of claims 1 to 14, **characterized in that** the binding of the analyte can be detected by the addition of further molecules provided with a detectable property.

16. Use of the method according to at least one of claims 1 to 15 for determining the binding properties of molecules which change their binding properties upon introduction of a detectable property.

17. Use of the method according to at least one of claims 1 to 16 for the identification of analytes.

## Revendications

1. Méthode de dosage par compétition destinée à déterminer la liaison des molécules, comprenant les étapes suivantes :
a) préparation d'une solution comportant au moins deux édifices moléculaires (A,B) entrant en interaction, qui sont composés chacun d'au moins 2 molécules différenciables ( A,A'; B, B'),
dans laquelle l'édifice moléculaire **B** ne contient que des molécules (B,B') qui ne présentent pas de propriété à détecter,
et de telle manière qu'au moins une molécule (B ou B') d'au moins un édifice moléculaire **B** ne se lie qu'à au moins une molécule (B ou B') de cet édifice moléculaire **B** ne présentant aucune propriété à détecter,
dans laquelle la liaison d'au moins deux molécules de la solution provoque la modification d'au moins une propriété à détecter,
b) détermination d'au moins une propriété à détecter, au cours de laquelle les édifices moléculaires sont des systèmes chimiques en équilibre de liaison, et les molécules d'un édifice moléculaire comportent au moins un ligand et/ou un dérivé de ligand et au moins un récepteur correspondant.

2. Méthode selon la revendication 1, **caractérisée en ce que**, pour déterminer l'influence de substances synthétiques ou biologiques sur les propriétés de liaison des molécules dans les édifices moléculaires, on ajoute une analyte à la solution.

3. Méthode selon la revendication 1 et/ou 2, **caractérisée en ce qu'**au moins une molécule (A) d'au moins un édifice moléculaire **A** se lie aussi bien à au moins une molécule (A') présentant une propriété à détecter de son édifice moléculaire **A** qu'à au moins une molécule (B', B) d'au moins l'autre édifice moléculaire B, qui ne présente pas de propriété à détecter.

4. Méthode selon au moins une des revendications de 1 à 3, **caractérisée en ce qu'**au moins une molécule (A') d'au moins un édifice moléculaire **A** présente une propriété à détecter et qu'elle se lie uniquement à au moins une molécule (A) de cet édifice moléculaire **A** présentant une propriété à détecter.

5. Méthode selon au moins une des revendications de 1 à 4, **caractérisée en ce que** les concentrations d'au moins une molécule (A) de l'édifice moléculaire **A**, qui se lie aussi bien à la au moins une molécule (A'), offrant une propriété à détecter, faisant partie de son édifice moléculaire **A**, qu'à au moins une molécule de l'autre édifice moléculaire **B** ne présentant pas ces propriétés (B'), et les concentrations de ces molécules (A', B') présentant ou non des propriétés à détecter sont harmonisées de façon à ce que ces au moins deux molécules (A', B') entrent en compétition pour la liaison à au moins une molécule (A) de l'édifice moléculaire **B**.

6. Méthode selon au moins une des revendications de 1 à 5, **caractérisée en ce que** la concentration d'au moins une molécule (B) d'un édifice moléculaire B, qui se lie uniquement à la au moins une molécule (B'), qui ne possède aucune propriété à détecter, est mesurée de manière à ce que l'action de compétition de la au moins une molécule (B'), qui ne présente aucune des propriétés à détecter, vis-à-vis d'au moins une molécule (A') qui présente une propriété à détecter, pour liaison à la au moins une molécule (A) de l'édifice moléculaire **A**, est neutralisée en partie ou entièrement.

7. Méthode selon au moins une des revendications de 1 à 6, **caractérisée en ce que** la liaison de la au moins une molécule (A'), qui présente une propriété à détecter, à la au moins une molécule (A), est indiquée par une modification de cette propriété.

8. Méthode selon au moins une des revendications de 1 à 7, **caractérisée en ce que** l'analyte se lie à la au moins une molécule (B) d'un édifice moléculaire **B**, qui se lie pour sa part seulement à des molécules n'offrant aucune propriété à détecter.

9. Méthode selon au moins une des revendications de 1 à 8, **caractérisée en ce que** la au moins une propriété détectable est engendrée par le marquage d'au moins une molécule avec un isotope radioactif, un isotope non radioactif, un chromophore, un fluorophore, un colloïde de métal noble, un biopolymère, un polymère synthétique, de la biotine, un peptide, une chaîne de résidus d'histidine, un enzyme, une molécule chimioluminescente, une molécule phosphorescente, une molécule qui peut être excitée pour fournir une électroluminescence, un chélate de terre rare ou une combinaison d'entre eux.

10. Méthode selon au moins une des revendications de 1 à 9, **caractérisée en ce que** la détermination de la au moins une propriété détectable s'effectue par des méthodes radiométriques et/ou des méthodes fluorimétriques.

11. Méthode selon au moins une des revendications de 1 à 10, **caractérisée en ce qu'**on détermine des paramètres à partir des propriétés détectables.

12. Méthode selon au moins une des revendications de 1 à 11, **caractérisée en ce que** les molécules sont des peptides, des protéines, des récepteurs couplés à une protéine G, des molécules d'ADN, des molécules d'ARN, des macromolécules.

13. Méthode selon au moins une des revendications de 1 à 12, **caractérisée en ce qu'**au moins une molécule de l'édifice moléculaire est liée à une surface.

14. Méthode selon la revendication 13, **caractérisée en ce que** les particules de support sont constituées d'oxydes de métal, de silicate, de colloïde de métal, d'halogénure d'argent, de sulfure d'arsenic, de sulfure de nickel, de sulfure de cobalt, de colloïde de carbone et/ou de polymère organique.

15. Méthode selon au moins une des revendications de 1 à 14, **caractérisée en ce que** la liaison de l'analyte peut être mise en évidence par l'ajout d'autres molécules dotées d'une propriété à détecter.

16. Utilisation de la méthode selon au moins une des revendications de 1 à 15 pour déterminer les propriétés de liaison de molécules qui modifient leur propriétés de liaison par la présentation d'une propriété à détecter.

17. Utilisation de la méthode selon au moins une des revendications de 1 à 16 pour l'identification des analytes.
